# EUROPEAN PATENT APPLICATION

(11) **EP 1 393 731 A1**
(43) Date of publication of application: **03.03.2004**
(21) Application number: 02724610.7
(22) Date of filing: 11.04.2002
(51) Int. Cl.: A61K 31/506, A61K 45/00, A61P 13/02, A61P 13/08, A61P 35/00

(54) **NOVEL USE OF ARYLETHENESULFONAMIDE DERIVATIVE**

(30) Priority: 13.04.2001 JP 2001114958
(71) Applicant: YAMANOUCHI PHARMACEUTICAL CO. LTD., Tokyo 103-8411 (JP)
(72) Inventor: FUJIMORI, Akira, Yamanouchi Pharmaceutical Co.,Ltd, Tsukuba-shi, Ibaraki 305-8585 (JP); YUYAMA, Hironori, Yamanouchi Pharmaceutical Co.Ltd, Tsukuba-shi, Ibaraki 305-8585 (JP); SANAGI, Masanao, Yamanouchi Pharmaceutical Co.,Ltd, Tsukuba-shi, Ibaraki 305-8585 (JP); KOAKUTSU, Akiko, Yamanouchi Pharmaceutical Co.,Ltd, Tsukuba-shi, Ibaraki 305-8585 (JP); YAMAMOTO, Nobuyuki, Yamanouchi Pharmaceut. Co.,Ltd, Itabashi-ku, Tokyo 174-8612 (JP)
(74) Representative: HOFFMANN EITLE
(86) International application number: PCT/JP2002/003624
(87) International publication number: WO 2002/083142

(57) **Abstract**

Novel use of N-(6-methoxy-5-(2-methoxyphenoxy)-2-(pyrimidin-2-yl)-pyrimidin-4-yl]-2-phenylethenesulfonamide or a pharmaceutically acceptable salt thereof. That is, a pharmaceutical composition for remedy of dysuria containing N-[6-methoxy-5-(2-methoxyphenoxy)-2-(pyrimidin-2-yl)-pyrimidin-4-yl]-2-phenylethenesulfonamide or a pharmaceutically acceptable salt thereof as an active ingredient.

## Description

### Technical Field of the Invention

The present invention relates to a novel utility of N-[6-methoxy-5-(2-methoxyphenoxy)-2-(pyrimidin-2-yl)-pyrimidin-4-yl]-2-phenylethenesulfonamide or a salt thereof. Specifically, the invention relates to a pharmaceutical composition for remedy of dysuria comprising N-[6-methoxy-5-(2-methoxyphenoxy)-2-(pyrimidin-2-yl)-pyrimidin-4-yl]-2-phenylethenesulfonamide or a salt thereof as an active ingredient.

### Background Art

Urinary dysfunction is classified into (1) dysuria referring to a symptom of hard excretion of urine in which an increase of difficulty of urination or residual urine is caused, ultimately leading to urinary retention and (2) urine storage disorder referring to a symptom mainly composed of symptoms such as pollakiuria and urinary incontinence, in which urine cannot be sufficiently stored in a bladder.

The dysuria that is one of urinary dysfunction is classified into one due to bladder function and one accompanying obstruction of a lower urinary tract depending on its cause; and the dysuria due to obstruction of a lower urinary tract is classified into one attributing to urethra and one attributing to prostate in adult males. As a representative example of the dysuria attributing to prostate, dysuria due to benign prostatic hypertrophy is enumerated. The dysuria due to benign prostatic hypertrophy is a disease causing urinary dysfunction due to compression by prostate of prostatic urethra accompanying hypertrophy of prostate (mechanical obstruction) or excessive contraction of prostatic smooth muscle (functional obstruction) due to an increase of α₁ receptors, etc.

On the other hand, the dysuria attributing to urethra is dysuria caused by urethral calculus or urethral constriction extending the whole of lower urinary.tract not limiting to prostatic urethra, and is distinguished from the dysuria due to hypertrophy of prostate or compression of prostatic urethra by excessive contraction of prostatic smooth muscle.

Endothelin (hereinafter referred to as "ET") is an endogenous physiologically active peptide consisting 21 amino acids, and it is known that three kinds of isopeptides of ET-1, ET-2 and ET-3, which are slightly different from each other with respect to amino acid sequence, are present. ET is bound to an ET receptor on a target cell membrane to reveal its physiologic activity, and it has been known up to date that at least two kinds of subtypes of ET_{A} and ET_{B} receptors are present as the ET receptor. The ET_{A} receptor exhibits a higher affinity with ET-1 and ET-2 than ET-3, and the ET_{B} receptor exhibits an equal affinity with ET-1, ET-2 and ET-3.

N-[6-Methoxy-5-(2-methoxyphenoxy)-2-(pyrimidin-2-yl)-pyrimidin-4 -yl]-2-phenylethenesulfonamide (hereinafter referred to as "Compound A") or a salt thereof is disclosed in International Patent Publication No. 97/22595, and an inhibitory action of binding of ET-1 to the ET_{A} receptor and an inhibitory action against vasoconstriction and vasopressor induced by ET-1 are specifically disclosed, but other actions are not specifically disclosed. On the other hand, as diseases to which ET is pointed out to possibly contribute are enumerated essential hypertension, pulmonary hypertension, hypertension induced by erythropoietin, hypertension induced by cyclosporin A, bronchial asthma, acute renal failure, chronic renal failure, glomerular nephritis, renal failure induced by cyclosporin, acute myocardial infarction, unstable angina, congestive heart failure, cerebrovascular spasm mostly after subarachnoid hemorrhage, cerebroischemic disturbance, urinary incontinence, benign prostatic hypertrophy, arteriosclerosis, Raynaud's syndrome, diabetic peripheral circulatory disturbance, diabetic renal diseases, preeclampsia, premature delivery, digestive ulcer, hepatic failure, rheumatism, restenosis after PTCA, chronic respiratory failure, chronic obstructive pulmonary diseases, cor pulmonale, acute respiratory failure, pulmonary edema, ischemic hepatic disturbance, adult respiratory distress syndrome, interstitial pneumonia, pulmonary fibrosis, glaucoma, osteoarthritis, chronic articular rheumatism, hepatic cirrhosis, inflammatory bowel diseases, and cancer.

However, dysuria, especially dysuria accompanying excessive contraction over the whole of lower urinary tract including prostate is not disclosed.

### Disclosure of the Invention

The present inventors made extensive and intensive investigations about possibility of remedy against specific diseases by Compound A or a salt thereof for the purpose of creating novel therapeutic drugs. As a result, it has been unexpectedly found that Compound A or a salt thereof is effective for improving dysuria accompanying urethral contraction of the whole of lower urinary tract induced by acceleration of ET-1 production, an aspect of which is not specifically disclosed in the foregoing International Patent Publication No. 97/22595. In particular, it has been found that Compound A or a salt thereof is not only effective against dysuria due to mechanical compression by hypertrophied prostate in benign prostatic hypertrophy or excessive contraction of prostatic smooth muscle but also actually effective against dysuria accompanying contraction of the whole of lower urinary tract by ET. Namely, it has been found that Compound A or a salt thereof is also effective against dysuria in the case where no compression by prostate of prostatic urethra is present.

The details are as follows.

It is reported that ET-1 is produced in human epithelial cells and prostatic cancer cells in addition to endothelial cells to cause contraction or proliferation of prostatic smooth muscle (*J. Clin. End. Metab*., 82(2), 508-513, 1997; *Gen. Pharmac*., 27(6), 1061-1065, 1996; and *Eur. J. Pharmacol.,* 349, 123-128, 1998). Further, it is reported that when ET-1 is compared with phenylephrine hydrochloride as an α₁ receptor stimulant with respect to contraction activity against prostate preparations, ET-1 causes more continuous prostatic contraction at a lower concentration (*J. Urol*., 158, 253-257, 1997). Compound A or a salt thereof exhibited an inhibitory action against contraction by ET-1 in extracted rabbit lower urinary tract preparations as shown in Test Example 1 described later.

Also, Compound A or a salt thereof exhibited a dose-dependent inhibitory action against an increase of prostatic urethral pressure due to local administration of ET-1 to anesthetized dogs as shown in Test Example 2 described later. In the anesthetized dogs, local administration of phenylephrine hydrochloride (2 µg/kg i.a.) and ET-1 (0.4 µg/kg i.a.) largely increased a prostatic urethral pressure (PUP) without largely affecting blood pressure and heart rate. When dose response was compared, phenylephrine hydrochloride was higher with respect to the maximum increase of PUP, but ET-1 exhibited an increase response of PUP from a lower concentration and continued it longer. This result is well consistent with the *in vitro* report in extracted dog prostate preparations (*J*. *Urol.,* 155, 1758-1761, 1996), but it is also reported that ET-1 causes contraction of the same degree as in phenylephrine hydrochloride in extracted human prostate preparations, an aspect of which is different from the case of dog (*J*. *Urol.,* 150, 495-499, 1993).

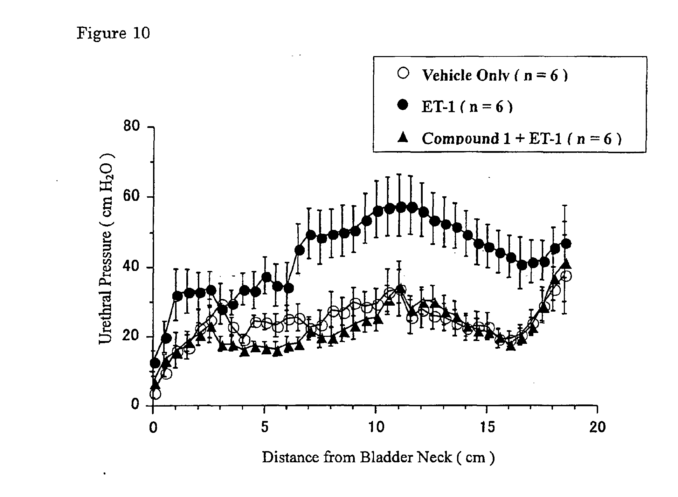

In addition, Compound A or a salt thereof exhibited an inhibitory action against an increase of urethral pressure of the whole of lower urinary tract including prostate urethra due to intravenous administration of ET-1 to anesthetized dogs as shown in Test Example 3 described later. In the anesthetized dogs, the intravenous administration of phenylephrine hydrochloride (30 µg/kg i.v.) largely increased mainly the urethral pressure of prostatic urethra in parallel to an increase of blood pressure, whereas the intravenous administration of ET-1 (1 µg/kg i.v.) increased the urethral pressure of lower urinary tract including urethra other than prostatic urethra without substantially affecting the blood pressure. As shown in Fig. 10, an increase of urethral pressure of the whole of lower urinary tract as induced by ET-1 was completely inhibited by pre-administration of a potassium salt of Compound A. That is, Compound A or a salt thereof also actually exhibited an inhibitory action against excessive contraction of the whole of lower urinary tract by ET not limiting to prostatic site.

Also, Compound A or a salt thereof exhibited an inhibitory action against an increase of urethral pressure of lower urinary tract of anesthetized dogs as induced by ET-1 in the presence of tamsulosin hydrochloride as an α₁ receptor blocker as shown in Test Example 4 described later. It is known that a contraction of isolated human prostate preparations by ET-1 is not inhibited by α₁ receptor blockers (*J. Urol*., 150, 495-499, 1993). In the anesthetized dogs, tamsulosin hydrochloride lowered a prostatic urethral pressure in the steady state, but ET-1 increased a urethral pressure of lower urinary tract including prostatic urethra even in the presence of tamsulosin hydrochloride. The increase of urethral pressure as induced by ET-1 in the presence of tamsulosin hydrochloride was remarkable in urethra other than prostatic urethra.

Incidentally, the potassium salt of Compound A improved an international prostate symptom score (I-PSS) of patients with prostatic cancer accompanied with urinary dysfunction by oral administration in clinical tests as shown in Test Example 5 described below.

In addition, it is reported that human prostatic endothelial cells have the ability to produce ET-1 (*J*. *Clin. End Metab.,* 82(2), 508-513, 1997); ET-1 proliferates prostatic smooth muscle cells via ET_{A} receptor (*Eur. J. Pharmacol*., 349, 123-128, 1998); human prostatic cancer cells have the ability to produce ET-1 (*Nat. Med*., 1(9), 944-949, 1995); ET-1 exhibits the ability to proliferate human prostatic cancer cells via ET_{A} receptor (*Cancer* *Res.,* 56, 663-668, 1996); the production of ET-1 is accelerated in prostatic cells of patients with benign prostatic hypertrophy (*Gen. Pharmac.,* 27(6), 1061-1065, 1996); and inflammatory cells have the ability to produce ET-1. Accordingly, when taking into account these reports in combination with the foregoing results that ET-1 contracts a lower urinary tract to increase a urethral pressure, it may be said that in prostatic diseases of accelerating the production of ET, such as prostatic cancer, benign prostatic hypertrophy, and prostatitis, Compound A or a salt thereof inhibits excessive contraction of the whole or lower urinary tract including prostate and/or hypertrophy of prostate and are effective for improving dysurias accompanying these diseases.

In addition, taking into account the report that the clinical result of α₁ receptor blockers reveals an improving rate of from 40 to 70 % in the measurement of urinary flow (*Rinsho To Kenkyu* (Clinics and Studies), 74(2), 50-53, 1997) and difference in mechanism of action from the α₁ receptor blockers shown by the invention, it may be said that Compound A or a salt thereof is a therapeutic agent for dysuria giving rise to an effect in combination with α₁ receptor blockers.

Accordingly, the active ingredient of the invention can improve not only the dysuria accompanying hypertrophy of prostate or compression by prostate of prostatic urethra by excessive contraction but also the dysuria due to contraction of the whole of lower urinary tract as induced by ET and can further improve the ET-induced dysuria due to prostatic diseases such as prostatic cancer, benign prostatic hypertrophy, and prostatitis.

Namely, the invention provides a pharmaceutical composition for remedy of dysuria containing Compound A or a pharmaceutically acceptable salt thereof as the active ingredient; a pharmaceutical composition for remedy of dysuria as a pharmaceutical composition for inhibiting ET-induced contraction of lower urinary tract; a pharmaceutical composition for remedy of dysuria as a pharmaceutical composition for inhibiting ET-induced contraction of lower urinary tract due to prostatic diseases; a pharmaceutical composition for remedy of dysuria as a pharmaceutical composition for inhibiting ET-induced contraction of lower urinary tract due to prostatic cancer and/or benign prostatic hypertrophy and/or prostatitis; or a pharmaceutical composition for remedy of dysuria that is characterized by being used in combination with α₁ receptor blockers.

Further, the invention relates to use of Compound A or a pharmaceutically acceptable salt thereof for production of a therapeutic agent of dysuria; a therapeutic agent of dysuria as a depressant of ET-induced contraction of lower urinary tract; a therapeutic agent of dysuria as a depressant of ET-induced contraction of lower urinary tract due to prostatic diseases; a therapeutic agent of dysuria as a depressant of ET-induced contraction of lower urinary tract due to prostatic cancer and/or benign prostatic hypertrophy and/or prostatitis; or a therapeutic agent of dysuria that is characterized by being used in combination with α₁ receptor blockers.

Moreover, the invention provides a method of remedy for dysuria including administering a patient with a therapeutically effective amount of Compound A or a pharmaceutically acceptable salt thereof; a method of remedy for dysuria as a method of inhibiting ET-induced contraction of lower urinary tract; a method of remedy for dysuria as a method of inhibiting ET-induced contraction of lower urinary tract due to prostatic diseases; a method of remedy for dysuria as a method of inhibiting ET-induced contraction of lower urinary tract due to prostatic cancer and/or benign prostatic hypertrophy and/or prostatitis; or a method of remedy for dysuria that is characterized by being carried out in combination with α₁ receptor blockers.

Incidentally, since the active ingredient of the invention is particularly excellent in oral absorbing property, it can be an excellent oral therapeutic drug.

The invention will be hereunder described in more detail.

In this description, the "dysuria" refers to a symptom of hard excretion of urine in which an increase of urinary dysfunction or residual urine is caused, ultimately leading to urinary retention, i.e., it means dysuria.

Further, the "inhibition of contraction of lower urinary tract" means inhibition of ET-induced contraction of the whole of lower urinary tract such as urethra including bladder base, prostate, and prostatic urethra.

Moreover, examples of α₁ receptor blockers that can be used in combination with the active ingredient of the invention include prazosin hydrochloride, terazosin hydrochloride, urapidil, tamsulosin hydrochloride, bunazosin hydrochloride, doxazosin, and naftopidil, with tamsulosin hydrochloride being particularly preferred.

The active ingredient of drug of the invention is Compound A or a pharmaceutically acceptable salt thereof. As such salts are enumerated salts as described in the foregoing International Patent Publication No. 97/22595. Specific examples include acid addition salts of inorganic acid such as hydrochloric acid, hydrobromic acid, hydroiodic acid, sulfuric acid, nitric acid, and phosphoric acid, or organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, and glutamic acid; salts of inorganic bases such as sodium, potassium, magnesium, calcium, and aluminum, or organic bases such as methylamine, ethylamine, ethanolamine, lysine, and ornithine; and ammonium salts, with potassium salts being particularly preferred.

Also, the active ingredient of the invention includes all of mixtures of various isomers and isolated isomers, hydrates and solvates thereof. There may be the case where the active ingredient of the invention has a crystal polymorphism, and the active ingredient of the invention includes all of such crystal polymorphisms.

These compounds are readily available by the production process as descried in the foregoing International Patent Publication No. 97/22595 or according to that production process.

The drug of the invention can be prepared as oral solid preparations, oral liquid preparations or injections using an organic or inorganic carrier, an excipient, and other additives suitable for oral or parenteral administration according to the customary manner. Since the active ingredient of the drug of the invention has excellent oral absorbing property, the drug of the invention is suitable for oral preparations. Oral solid preparations that patients can easily take themselves and are convenient in storage and conveyance are the most preferable.

Examples of oral solid preparations include tablets, powders, fine granules, granules, capsules, pills, and sustained release products. In such solid compositions, at least one active substance is mixed with at least one inert diluent such as lactose, mannitol, glucose, hydroxypropyl cellulose, microcrystalline cellulose, starch, corn starch, polyvinylpyrrolidone, and magnesium metasilicate aluminate. The composition may contain additives other than inert diluents such as binders such as hydroxypropyl cellulose and hydroxypropylmethyl cellulose; lubricants such as magnesium stearate, polyethylene glycol, starch, and talc; disintegrating agents such as cellulose calcium glycolate and carmellose calcium; stabilizers such as lactose; dissolution aids such as glutamic acid and aspartic acid; plasticizers such as polyethylene glycol; coloring agents such as titanium oxide, talc, and yellow iron oxide according to the customary manner. If desired, the tablets or pills may be coated with a sugar coating such as sugar, gelatin, agar, pectin, hydroxypropyl cellulose, and hydroxypropylmethyl cellulose phthalate, or a gastric-soluble or intestinal soluble film.

The oral liquid preparations include pharmaceutically acceptable emulsions, solutions, suspensions, syrups and elixirs and contain a generally employed inert diluent such as purified water and ethanol. This composition may contain an auxiliary agent such as wetting agents and suspending agents, a sweetener, a flavor, an aromatic, or an antiseptic, in addition to the inert diluent.

The injections such as intravenous injections, intramuscular injections, and subcutaneous injections include sterile aqueous or non-aqueous solutions, suspensions and emulsions. Examples of diluents for aqueous solutions or suspensions include distilled water and physiological saline for injections. Examples of diluents for non-aqueous solutions or suspensions include propylene glycol, polyethylene glycol, vegetable oils such as olive oil, alcohols such as ethanol, and Polysolvate 80. Such a composition may further contain auxiliary agents such as an antiseptic, a wetting agent, an emulsifier, a dispersant, a stabilizer such as lactose, and a dissolution aid such as glutamic acid and aspartic acid. These compositions are sterilized by, for example, filtration through a bacteria-holding filter, compounding with an anti-bacterial agent, or irradiation. Further, these can be used by producing a sterile solid composition and dissolving it in sterile water or a sterile solvent for injection before use.

The dose of the active ingredient compound of the invention is properly determined depending on the individual case while taking into account the administration route, symptom of a patient, age of the subject to be administered, sex, etc. In the case of oral administration, the active ingredient can be usually administered in a dose of from about 0.1 to 300 mg/day, and preferably from 1 to 60 mg/day per adult once or dividedly twice.

Incidentally, the drug of the invention can be used in combination with other drugs that are used for remedy of urinary dysfunction accompanying prostatic diseases simultaneously or after elapsing for a while. Examples of drugs that can be used in combination with the drug of the invention include α₁ receptor blockers such as prazosin hydrochloride, terazosin hydrochloride, urapidil, tamsulosin hydrochloride, bunazosin hydrochloride, doxazosin, and naftopidil; anticholinergics and prostatic smooth muscle relaxants such as propiverine hydrochloride, flavoxate hydrochloride, oxybutynin hydrochloride, tolterodine, trospium, and hyoscyamine; β receptor agonists such as clenbuterol hydrochloride; quaternary ammonium salt preparations such as bethanechol chloride and distigmine bromide; antidiuretic hormones such as desmopressin; antidepressants and minor tranquilizer such as amitriptyline hydrochloride and imipramine; *kampo* (Chinese) medicines such as *hachimijiogan*; amino acid combination products such as L-glutamic acid, L-alanine, and aminoacetic acid; pollen preparations; and vegetable extract combination products.

Further, the drug of the invention can be used in combination with other drugs used for remedy of prostatic cancer or prostatitis simultaneously or after elapsing for a while. Examples of drugs that can be used in combination with the drug of the invention include anti-malignant tumor agents such as ifosfamide and tegafur-uracil; follicle hormones such as ethynyl estradiol; adrenocortical hormones such as hydrocortisone and prednisolone; progesterone hormones such as chlormadinone acetate; LH-RH agonists such as goserelin acetate and leuprorelin acetate; anti-malignant tumor platinum complex compunds such as cisplatin; anti-androgen agents such as flutamide, bicalutamide, allylestrenol, and oxendolone; therapeutic agents for prostatic cancer such as estramustine phosphate sodium and fosfestrol; anti-tumor antibiotics such as peplomycin sulfate; pyridonecarboxylic acid antibacterial agents such as norfloxacin, tosufloxacin tosilate, levofloxacin, and ciprofloxacin hydrochloride; and cephem based antibiotic preparations such as ceftazidime.

### Brief Description of the Drawings

Fig. 1 is a graph showing contraction rates by ET-1, phenylephrine hydrochloride, sarafotoxin S6c (hereinafter referred to as "S6c"), and acetylcholine at respective concentrations in isolated rabbit a) prostate preparations, b) urethra preparations and c) bladder base preparations.
Fig. 2 is a graph showing inhibitory effects of a potassium salt of Compound A (hereinafter referred to as "Compound 1") against contraction of ET-1 in isolated rabbit a) prostate preparations, b) urethra preparations and c) bladder base preparations.
Fig. 3 is a graph showing a change of heart rate, a change of blood pressure and an increasing action of prostatic urethral pressure by local administration of ET-1 and phenylephrine hydrochloride in anesthetized dog.
Fig. 4 is a graph showing an increasing action of prostatic urethral pressure by local administration of ET-1 and phenylephrine hydrochloride in anesthetized dog.
Fig. 5 is a graph showing an inhibitory effect of Compound 1 against an increase of prostatic urethral pressure by local administration of ET-1 in anesthetized dog.
Fig. 6 is a graph showing a typical change of urethral pressure with the catheter drawing measurement in anesthetized dog.
Fig. 7 is a graph showing an increasing action of urethral pressure by intravenous administration of ET-1 in anesthetized dog.
Fig. 8 is a graph showing an increasing action of urethral pressure by intravenous administration of phenylephrine hydrochloride anesthetized dog.
Fig. 9 is a graph showing an increasing action of blood pressure and an increasing action of prostatic urethral pressure by intravenous administration of ET-1 and phenylephrine hydrochloride in anesthetized dog.
Fig. 10 is a graph showing an inhibitory effect of Compound 1 against an increase of urethral pressure by intravenous administration of ET-1 in anesthetized dog.
Fig. 11 is a graph showing an inhibitory effect of Compound 1 against an ET-1 induced increase of urethral pressure in anesthetized dog in the presence of an α₁ receptor blocker.

### Best Mode For Carrying Out The Invention

The invention will be hereunder described in detail with reference to the following Examples and Test Examples, but it should not be construed that the invention is limited to these Examples. Incidentally, Compound 1 as used in the following Examples and Test Examples means a potassium salt of Compound A.

### Example 1: Capsules

**(Table 1)**

| Component | 2mg Capsule | 5mg Capsule | 10mg Capsule |
|---|---|---|---|
| Compound 1 | 2.0 mg | 5.0 mg | 10.0 mg |
| Lactose | 298.0 mg | 295.0 mg | 290.0 mg |
| Total | 300.0 mg | 300.0 mg | 300.0 mg |

The components as shown in Table 1 are mixed and filled in a capsule to give a capsule preparation.

### Test Example 1: Inhibitory effect against ET-1 induced contraction in isolated rabbit lower urinary tract preparation

### (Method)

Male Japanese white rabbits (from 3 to 5 kg, available from Kitayama Labes Co., Ltd.) were provided for experiments. Prostate, urethra and bladder were isolated from the rabbit to prepare ring preparations of smooth muscle. With respect to the bladder, a dorsal region of the bladder base was used as preparation. Each of the preparations was suspended in an organ bath filled with a Krebs-Henselit solution at 37 °C, into which was then aerated a 95 % O₂-5 % CO₂ gas. A resting tension was subjected as 1 g, and a tension change was isometrically measured. Incidentally, the organ bath for preventing adsorption of ET-1 was previously subjected to silicon treatment. After elapse of an equilibrium period for 60 minutes, the preparation was contracted twice by 10 µM phenylephrine hydrochloride to be stabilized the responsibility, and a main experiment was started.

ET-1 (from 0.1 to 1,000 nM), phenylephrine hydrochloride (from 0.01 to 100 µM), S6c (from 1 to 1,000 nM), or acetylcholine (from 0.01 to 1,000 µM) was cumulatively added to the organ bath to obtain a concentration-response curve. Further, after treating with Compound 1 (from 0.1 to 10 µM) or a solvent (distilled water) for 30 minutes, ET-1 (from 0.1 to 1,000 nM) was cumulatively added to obtain a concentration-response curve. The contraction response to each agonists was expressed while defining the contraction by phenylephrine hydrochloride (10 µM) as obtained immediately therebefore as 100 %.

A concentration (EC₅₀) at which 50 % of the maximum contraction induced by ET-1 was determined by means of regression analysis; a concentration ratio of EC₅₀ of the concentration-response curve of ET-1 in the presence of Compound 1 to EC₅₀ of the concentration-response curve of ET-1 under the solvent treatment was determined; and a pA₂ and a slope were determined by the Schild's method from the obtained concentration ratio and the concentration of Compound 1 used.

### (Results)

### (1) Comparison of concentration-response of each agonist:

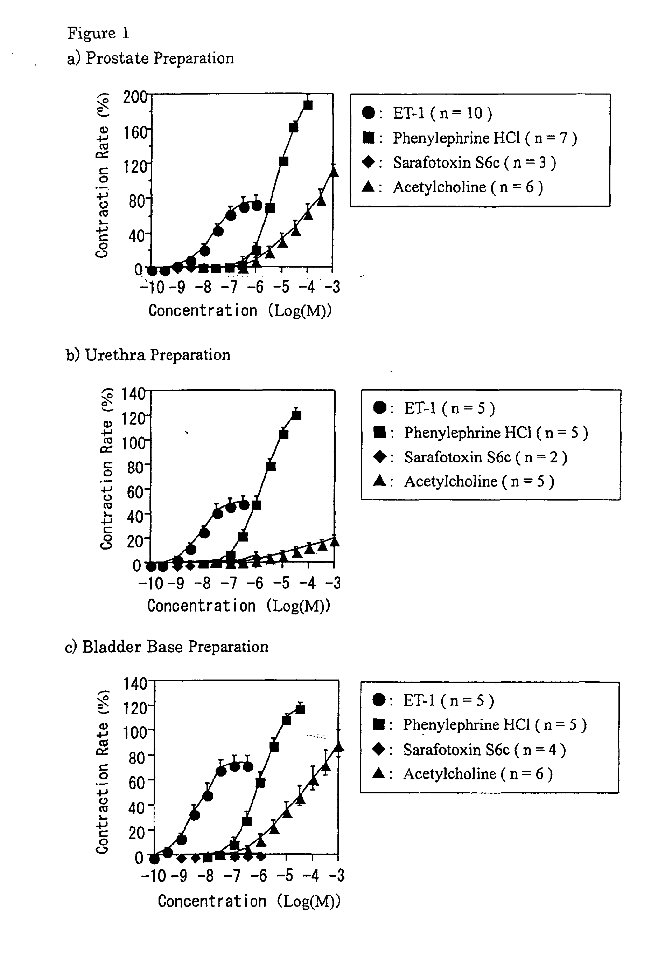

As shown in Fig. 1, ET-1, phenylephrine hydrochloride and acetylcholine contracted isolated rabbit prostate, urethra and bladder base preparations depending on the concentration. Acetylcholine did not reach the maximum contraction even at 1,000 µM. The maximum contraction of ET-1 against these three kinds of preparations was from 35 to 65 % of the contraction by phenylephrine hydrochloride, but the sensitivity (EC₅₀) was about 200 to 300 times higher than that of phenylephrine hydrochloride. Further, in these preparations, contraction by S6c as an ET_{B} receptor agonist was not substantially observed in these preparations, indicating almost the absence of ET_{B} receptor-mediated contraction in these tissues.

### (2) Effect of Compound 1 against ET-1 induced contraction:

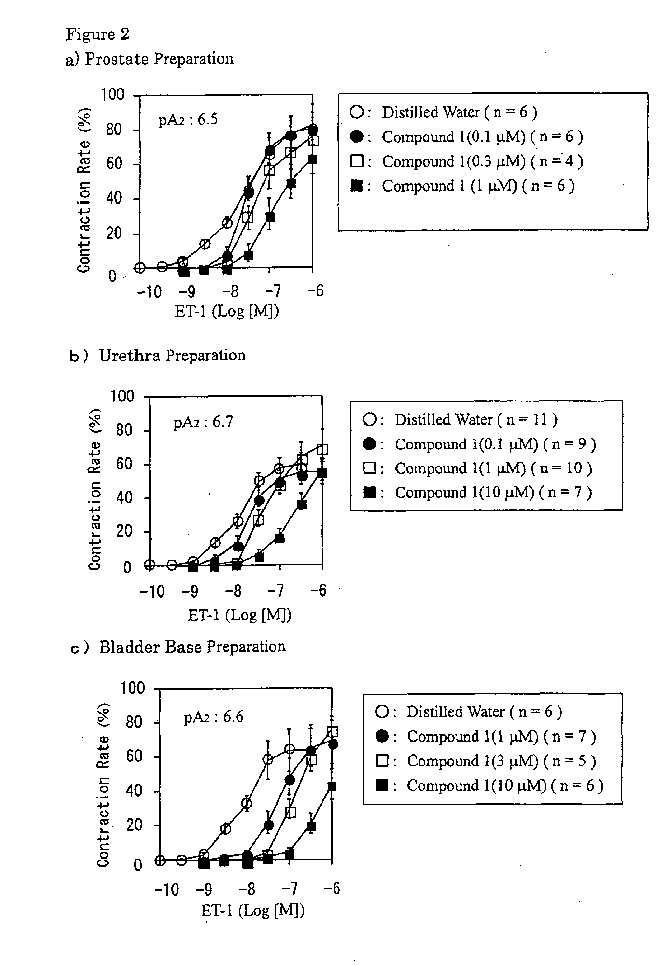

As shown in Fig. 2, in isolated rabbit prostate, urethra and bladder base preparations, Compound 1 shifted rightward the concentration-response curve of ET-1 induced contraction depending on the concentration with a pA₂ value of 6.5 (slope: 1.54), 6.7 (slope: 0.82) and 6.6 (slope: 1.32), respectively.

### Test Example 2: Increasing action of prostatic urethral pressure due to intraarterial local administration of ET-1 in anesthetized dog

### (Method)

Male beagle dogs (body weight: from 8 to 15 kg) were subjected to artificial ventilation under the anesthesia with pentobarbital, a thermodilution catheter (5Fr, 4 lumens) was inserted into a left femoral artery for measurement of blood pressure and heart rate and for administration of ET-1 or phenylephrine hydrochloride, and a 7Fr catheter was inserted into a left femoral vein for administration of Compound 1. Further, a catheter for measurement of urethral pressure (thermodilution catheter (6Fr, 4 lumens)) having a balloon purged with water was inserted from an external urethral meatus toward urinary bladder. After waiting for stabilization of hemodynamics following the operation, the balloon was fixed to the prostatic urethra and expanded, and a prostatic urethral pressure (PUP) was measured from a change in pressure within the balloon. The prostatic urethra was previously contracted twice or more with phenylephrine hydrochloride (8 µg/kg i.a.), and it was confirmed that a sufficient response was obtained, following the stabilization periods, an action of drug was studied.

In studying the action of ET-1 and phenylephrine hydrochloride, the drug were administered as an intra-arterial bolus injection just above the branches of an external iliac artery using the catheter inserted from the left femoral artery and exerted to a dominant region of internal iliac artery. ET-1 (from 0.006 to 0.8 µg/kg i.a., molar concentration: from 0.0025 to 0.321 nmol/kg i.a.) and phenylephrine hydrochloride (from 0.06 to 8 µg/kg i.a.; molar concentration: from 0.307 to 39.3 nmol/kg i.a.) were locally administered, thereby comparing the dose-response curve of an increasing action of PUP by the drugs. At this time, a right femoral artery was obstructed such that the drug did not flow out into a counterpart of external iliac artery.

Compound 1 (from 0.1 to 3 mg/kg) was intravenously administered five minutes before, and ET-1 (0.2 µg/kg) was administered then intra-arterially to measure PUP, thereby studying dose-dependency of antagonism of Compound 1 against the increasing action of PUP by ET-1. As the concentration of ET-1, was used a dose of 0.2 µg/kg i.a. at which ET-1 sufficiently increased the urethral pressure without affecting blood pressure, etc. and could be repeatedly administered in a preliminary study.

In the experiments, dogs in which a degree of increase of PUP was observed 40 cmH₂O or more in the pre-contraction with phenylephrine hydrochloride (8 µg/kg i.a.) were used. The results of each of the experiments were evaluated with respect to a degree of increase from PUP immediately before the drug administration to PUP after the drug administration and expressed in terms of "mean ± standard error". An EC₅₀ value of the dose-response curve of 8 µg/kg i.a. of phenylephrine hydrochloride and an IC₅₀ value of an inhibitory effect of Compound 1 were calculated by the logistic regression method.

### (Results)

### (1) Increasing action of prostatic urethral pressure due to local administration of ET-1 and phenylephrine hydrochloride:

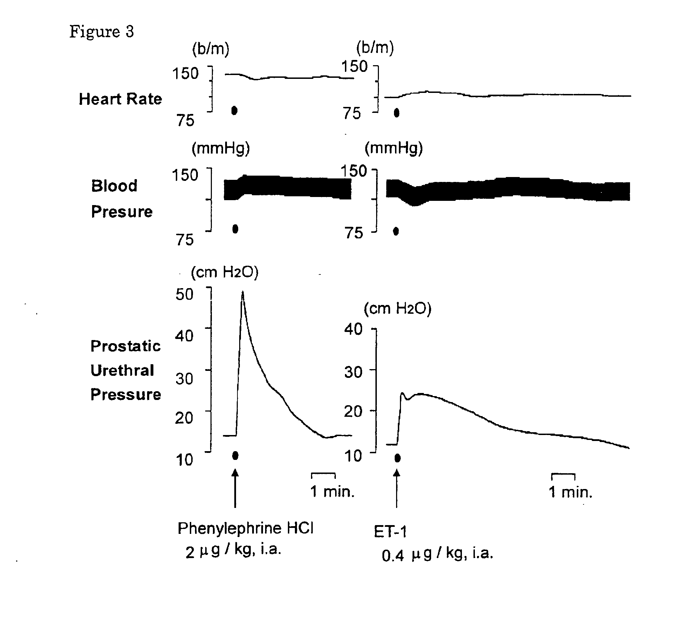

Fig. 3 shows a typical example when ET-1 (0.4 µg/kg i.a.) or phenylephrine hydrochloride (2 µg/kg i.a.) was intra-arterially administered in a dominant region of internal iliac artery. With respect to the blood pressure and heart rate, phenylephrine hydrochloride exhibited a tendency to increase of blood pressure and reduction of heart rate, and ET-1 exhibited a tendency to transient reduction of blood pressure and transient increase of heart rate, but any of them did not bring about large influences. On the other hand, with respect to PUP, both phenylephrine hydrochloride and ET-1 markedly increased PUP. The phenylephrine hydrochloride administration was higher about twice with respect to the degree of increase of PUP, and the ET-1 administration was longer about 3 times with respect to the duration of increase response of PUP.

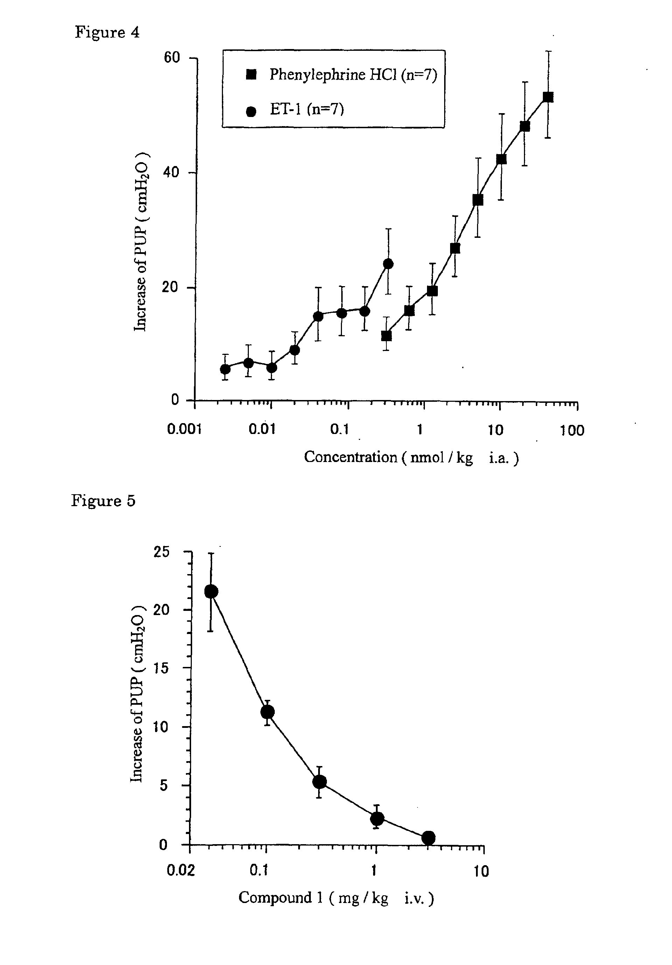

As shown in Fig. 4, both of ET-1 (from 0.006 to 0.8 µg/kg i.a.) and phenylephrine hydrochloride (from 0.06 to 8 µg/kg i.a.) increased PUP in a concentration dependent manner. The EC₅₀ value reduced into molar concentration because of a difference of molecular weight was 0.08 ± 0.03 nmol/kg for ET-1 and 7.47 ± 2.82 nmol/kg for phenylephrine hydrochloride, respectively, and therefore, it was considered that ET-1 revealed the increasing action of prostatic urethral pressure at a concentration of about 100 times lower than phenylephrine hydrochloride. Further, the maximum response of ET-1 was 43.9 ± 5.2 % of that of phenylephrine hydrochloride.

### (2) Inhibitory effect of Compound 1 against ET-1 induced increase of prostatic urethral pressure:

As shown in Fig. 5, the intravenous administration of Compound 1 (from 0.03 to 3 mg/kg i.v.) concentration-dependently inhibited the increase of PUP by ET-1 (0.2 µg/kg i.a.) in anesthetized dogs, and its IC₅₀ value was 0.13 ± 0.07 mg/kg.

### Test Example 3: Increasing action of urethral pressure due to intravenous administration of ET-1 in anesthetized dog

### (Method)

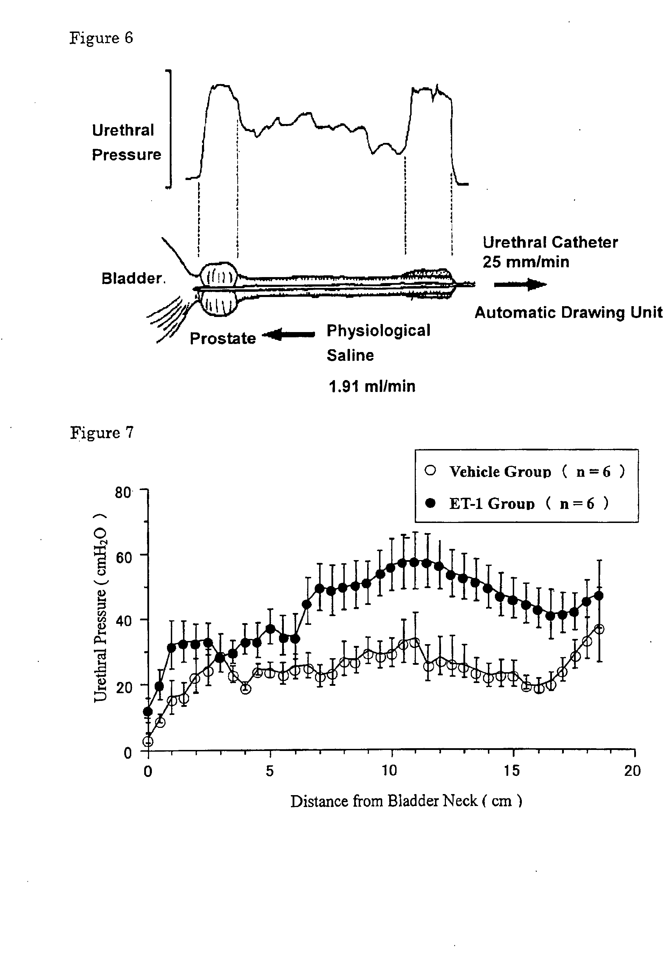

Male beagle dogs (body weight: from 8 to 13 kg) were subjected to artificial ventilation under the anesthesia with pentobarbital, a catheter was inserted into a left femoral artery for measurement of blood pressure and heart rate and a left femoral vein for administration of drug. A catheter for measurement of urethral pressure (from 8 to 10Fr, 2 holes, manufactured by Create Medic Co., Ltd.) was inserted from an external urethral meatus toward urinary bladder. An automatic syringe pump was connected with one end of the catheter, and a pressure transducer was connected with the other end thereof. After elapse of a stabilization period following the operation, physiological saline was poured using the automatic syringe pump into the urethra through the catheter for measurement of urethral pressure at a flow rate of 1.91 mL/min. At the same time, by drawing out the catheter from the urinary bladder into the external urethral meatus at a rate of 25 mm/min by an automatic drawing unit, the pressure was measured and recorded throughout urethra as a urethral pressure profile (UPP) as shown in Fig. 6.

Study of the action of ET-1 and phenylephrine hydrochloride against UPP was evaluated by measuring the urethral pressure after intravenous administration of ET-1 (1 µg/kg) or phenylephrine hydrochloride (30 µg/kg). In the blood pressure-response by ET-1, since the pressure increased after a transient reduction of pressure, measurement of the urethral pressure was started about 3 minutes after the administration. On the other hand, as phenylephrine hydrochloride caused an increase of pressure immediately after the administration, measurement of the urethral pressure was started immediately after the administration.

Thirty minutes after confirming recovery of the urethral pressure to a steady state, Compound 1 (1 mg/kg) was further intravenously administered. Three minutes after the administration, ET-1 (1 µg/kg) was again intravenously administered, and an action against the urethral pressure was studied similarly to the time of single administration.

### (Results)

### (1) Increasing action of urethral pressure due to intravenous administration of ET-1 and phenylephrine hydrochloride:

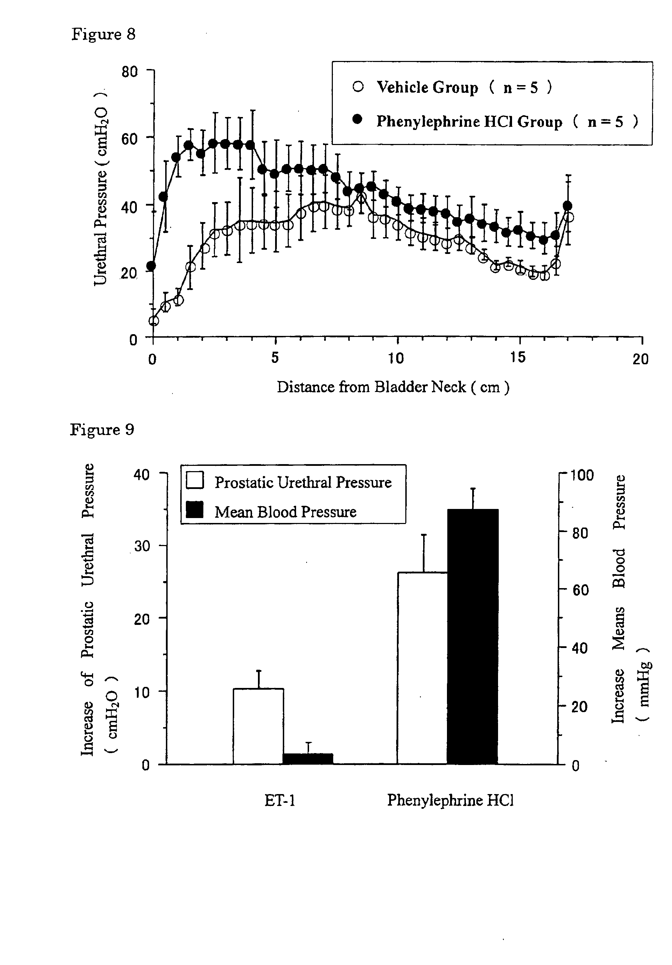

As shown in Figs. 7 and 9, in the anesthetized dogs, ET-1 (1 µg/kg i.v.) brought about a remarkable increase of the pressure throughout urethra other than prostate in addition to an increase of the urethral pressure of prostate, without substantially affecting the mean blood pressure.

On the other hand, as shown in Figs. 8 and 9, phenylephrine hydrochloride (30 µg/kg i.v.) brought an increase of mean blood pressure and a remarkable increase of the urethral pressure of prostate especially in UPP. (2) Inhibitory action of Compound 1 against an increase of urethral pressure as induced by ET-1:

As shown in Fig. 10, an increasing action of UPP due to intravenous administration of ET-1 was completely inhibited by pre-administration of Compound 1 (1 mg/kg i.v.).

### Test Example 4: Increasing action of urethral pressure as induced by ET-1 in the presence of α₁ receptor blocker in anesthetized dog

### (Method)

A urethral pressure profile (UPP) was recorded according to the same operation method as in Test Example 3. An action of ET-1 in a urethral pressure of anesthetized dog in the presence of tamsulosin hydrochloride as an α₁ receptor blocker was studied in the following procedures.

Tamsulosin hydrochloride (10 µg/kg i.v.) was intravenously administered to confirm its reducing action of urethral pressure in UPP. Thirty minutes thereafter, the same amount of tamsulosin hydrochloride (10 µg/kg i.v.) was again intravenously administered. About three minutes after the administration, ET-1 (1 µg/kg) was intravenously administered to study an action of ET-1 in the urethral pressure together with tamsulosin hydrochloride.

Thirty minutes after confirming recovery of the urethral pressure to a steady state, tamsulosin hydrochloride (10 µg/kg i.v.) and Compound 1 (1 mg/kg i.v.) were further intravenously administered. Three minutes after the administration, ET-1 (1 µg/kg i.v.) was intravenously administered, and an effect of Compound 1 against an ET-1 induced increase of urethral pressure in the presence of tamsulosin hydrochloride was studied.

### (Results)

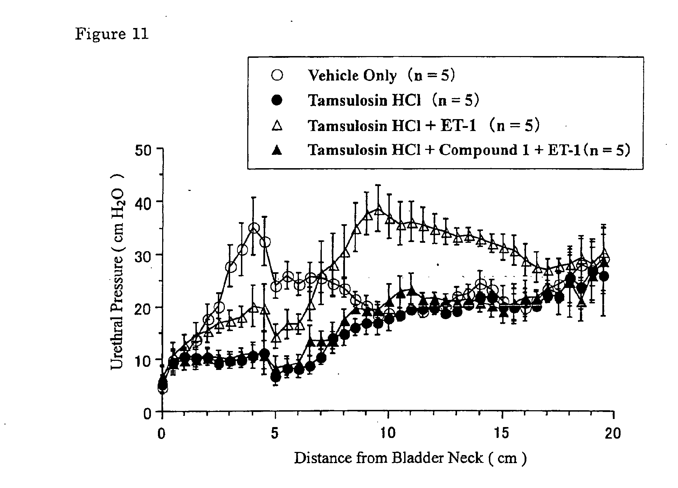

As shown in Fig. 11, tamsulosin hydrochloride (10 µg/kg i.v.) reduced the prostatic urethral pressure in anesthetized dogs. ET-1 (1 µg/kg i.v.) increased the urethral pressure of the whole of lower urinary tract including prostate even in the presence of tamsulosin hydrochloride without affecting the blood pressure. An increase of the urethral pressure was remarkable in urethra other than prostate. An increasing action of UPP by ET-1 was completely inhibited by pre-administration of Compound 1 (1 mg/kg i.v.) in the presence of tamsulosin hydrochloride.

### Test Example 5: Change of I-PSS during repeated oral administration of Compound 1 to patients with prostatic cancer accompanied with urinary dysfunction

### (Method)

Compound 1 (from 2 to 240 mg) was repeatedly orally administered to eighteen subjects of patients with prostatic cancer for 4 weeks, to measure an international prostate symptom score (I-PSS) before and after the administration. Ten subjects of patients with prostatic cancer having an I-PSS of 8 or more before the administration of Compound 1 were analyzed as a case accompanied with urinary dysfunction.

### (Results)

**(Table 2)**

| | Change of I-PSS (international prostate symptom score) | | |
|---|---|---|---|
| | Improved | Not changed | Deteriorated |
| Number of cases / total number of cases | 8/10 | 2/10 | 0/10 |
| Response rate | 80% | 20% | 0% |

As shown in Table 2, Compound 1 improved I-PSS of patients with prostatic cancer accompanied with urinary dysfunction. In the improved cases, a reduction rate of I-PSS due to administration of Compound 1 was calculated. As a result, the reduction rate of I-PSS was 31.7 ± 7.9 %.

It was confirmed from the results of Test Example 1 that Compound 1 exhibits an inhibitory effect against the ET-induced contraction of lower urinary tract *in vitro*.

It was confirmed from the results of Test Example 2 that Compound 1 exhibits an inhibitory effect against the ET-induced increase of prostatic urethral pressure *in vivo*.

It was confirmed from the results of Test Example 3 that Compound 1 exhibits an inhibitory effect against the ET-induced increase of prostatic urethral pressure of the whole of lower urinary tract not limiting to prostate and less affects the blood pressure *in vivo*.

From these inhibitory effects, it can be expected that Compound 1 exhibits an improvement effect in early subjective symptom against dysuria due to an increase of urethral pressure of the whole of lower urinary tract including prostatic urethra to be caused in prostatic diseases such as prostatic cancer, benign prostatic hypertrophy, and prostatitis, wherein the production system of ET-1 is accelerated.

On the other hand, although it is known that ET-1 causes proliferation of prostatic smooth muscle cells and prostatic cancer cells (*Eur. J. Pharmacol.,* 349, 123-128, 1998 and *Cancer Res*., 56, 663-668, 1996), there is a possibility that long-term administration of Compound 1 is also effective against hypertrophy of prostate.

It was confirmed from the results of Test Example 4 that Compound 1 gives rise to an effective improving effect of dysuria by a combined use of an α₁ receptor blocker as an existing therapeutic drug for dysuria. That is, since control of a sympathetic nerve (α₁ receptor) system and control of an ET system participate in urethral constriction with a different mechanism, it can be expected that the combined use gives rise to a more effective improvement against dysuria due to urethral constriction.

In addition, it was confirmed from the results of Test Example 5 that Compound 1 gives rise to a sufficient remedy effect against urinary dysfunction of a patient with prostatic cancer accompanied with dysuria by oral administration, too.

### Industrial Applicability

According to the invention, it is possible to provide an excellent pharmaceutical composition for remedy of dysuria.

Specifically, it is possible to provide a pharmaceutical composition for remedy of dysuria as a pharmaceutical composition for inhibition of ET-induced contraction of lower urinary tract and further to provide a pharmaceutical composition for remedy of dysuria accompanying prostatic diseases wherein acceleration of ET is found, especially prostatic cancer, benign prostatic hypertrophy, and prostatitis.

Further, according to the invention, it is possible to provide a pharmaceutical composition for improving pollakiuria accompanying dysuria due to prostatic diseases and a pharmaceutical composition for reducing residual urine accompanying dysuria due to prostatic diseases, and examples of the foregoing dysuria due to prostatic diseases include dysuria accompanying prostatic cancer and/or benign prostatic hypertrophy and/or prostatitis and ET-induced dysuria accompanying prostatic cancer and/or benign prostatic hypertrophy and/or prostatitis.

## Claims

1. A pharmaceutical composition for remedy of dysuria containing N-[6-methoxy-5-(2-methoxyphenoxy)-2-(pyrimidin-2-yl)pyrimidin-4-yl]-2-phenylethenesulfonamide or a pharmaceutically acceptable salt thereof as the active ingredient.

2. The pharmaceutical composition for remedy of dysuria according to claim 1, which is a pharmaceutical composition for inhibition of endothelin-induced contraction of lower urinary tract.

3. The pharmaceutical composition for remedy of dysuria according to claim 2, which is **characterized in that** the endothelin induction is endothelin induction due to prostatic diseases.

4. The pharmaceutical composition for remedy of dysuria according to claim 3, wherein the prostatic diseases are at least one prostatic disease selected from the group consisting of prostatic cancer, benign prostatic hypertrophy, and prostatitis.

5. The pharmaceutical composition for remedy of dysuria according to any one of claims 1 to 4, which is **characterized by** being used in combination with an α₁ receptor blocker.

6. The pharmaceutical composition for remedy of dysuria according to any one of claims 1 to 4, which is an oral administration drug.

7. Use of N-[6-methoxy-5-(2-methoxyphenoxy)-2-(pyrimidin-2-yl) pyrimidin-4-yl]-2-phenylethenesulfonamide or a pharmaceutically acceptable salt thereof for the production of therapeutic agent for dysuria.

8. The use according to claim 7, wherein the therapeutic agent for dysuria is a therapeutic agent for dysuria as a depressant for endothelin-induced contraction of lower urinary tract.

9. The use according to claim 8, wherein the endothelin induction is endothelin induction due to prostatic diseases.

10. The use according to claim 9, wherein the prostatic diseases are at least one prostatic disease selected from the group consisting of prostatic cancer, benign prostatic hypertrophy, and prostatitis.

11. The use according to any one of claims 7 to 10, which is **characterized by** being used in combination with an α₁ receptor blocker.

12. The use according to any one of claims 7 to 11, which is an oral administration drug.

13. A method of remedy for dysuria including administering a therapeutically effective amount of N-[6-methoxy-5-(2-methoxyphenoxy)-2-(pyrimidin-2-yl)-pyrimidin-4-yl]-2-phenylethenesul fonamide or a pharmaceutically acceptable salt thereof to a patient.

14. The method of remedy for dysuria according to claim 13, which is a method of inhibiting endothelin-induced contraction of lower urinary tract.

15. The method of remedy for dysuria according to claim 14, which is **characterized in that** the endothelin induction is endothelin induction due to prostatic diseases.

16. The method of remedy for dysuria according to claim 15, wherein the prostatic diseases are at least one prostatic disease selected from the group consisting of prostatic cancer, benign prostatic hypertrophy, and prostatitis.

17. The method of remedy for dysuria according to any one of claims 13 to 16, which is **characterized by** being used in combination with an α₁ receptor blocker.

18. The method of remedy for dysuria according to any one of claims 13 to 17, which is oral administration.
